(19) Europäisches Patentamt
European Patent Office
Office européen des brevets

(11) Publication number: **0 254 989 B1**

(12) **EUROPEAN PATENT SPECIFICATION**

(45) Date of publication of the patent specification: **26.09.90**

(51) Int. Cl.[5]: **C07C 215/46**, C07C 217/56, C07D 209/08, C07D 333/54, C07D 307/78, C07D 333/58, C07D 231/56, C07D 249/18, C07D 311/58, C07D 471/04

(21) Application number: **87110341.2**

(22) Date of filing: **17.07.87**

(54) **Substituted 2-Aminotetralins.**

(30) Priority: **28.07.86 US 891223**

(43) Date of publication of application: **03.02.88 Bulletin 88/5**

(45) Publication of the grant of the patent: **26.09.90 Bulletin 90/39**

(84) Designated Contracting States: **AT BE CH DE ES FR GB GR IT LI LU NL SE**

(56) References cited:
**EP-A- 0 163 458**
**EP-A- 0 168 505**

(73) Proprietor: **Whitby Research Incorporated, 1001 Health Sciences Road West, Irvine, CA 92715(US)**

(72) Inventor: **Horn, Alan S., Noordwijkweg 3A, NL-9804 RA Noordhorn (GR)(NL)**

(74) Representative: **Patentanwälte Kohler - Schwindling - Späth, Hohentwielstrasse 41, D-7000 Stuttgart 1(DE)**

**EP 0 254 989 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

#### Field of the Invention

The invention relates generally to substituted 2-aminotetralins and to processes for preparing such compounds. More particularly, the invention relates to compounds for therapeutic use, in particular in treating disorders of the central nervous, cardiovascular and endocrine systems. The compounds of this invention are also useful for alleviating glaucoma in mammals.

#### Background of the Prior Art

It is known that various hydroxylated 2-aminotetralins of the general formula

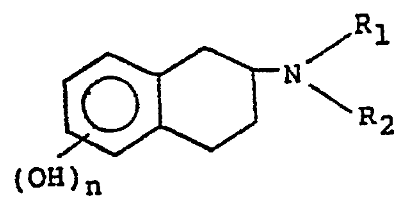

where $R_1$ and $R_2$ are saturated alkyl groups and n is 1 or 2, are dopamine receptor agonists (McDermed et al., J. Med. Chem. 18, 362 (1975); Feenstra et al., Arch. Pharmacol. 313, 213 (1980).

It is also known that certain dopaminergic compounds can lower intraocular pressure in various mammals. For example, it has been suggested that bromocriptine may lower intraocular pressure in man. (See The Lancet, February 4, 1984, "Bromocriptine Eyedrops Lower Intraocular Pressure without Affecting Prolactin Levels.", by Mekki, et al. at pages 287-288.)

Similarly, bromocriptine, as well as lergotrile and pergolide has been shown to lower the intraocular pressure of rabbits and the latter two compounds also lowered the intraocular pressure of monkeys. (see Potter, D.E. and Burke, J.A. (1982/1983), "Effects of Ergoline Derivatives on Intraocular Pressure and Iris Function in Rabbits and Monkeys", Curr. Eye res. 2, 281-288 and Potter, D.E., Burke, J.A. and Chang, F.W. (1984), "Ocular Hypotensive Action of Ergoline Derivatives in Rabbits: Effects of Sympathectomy and Domperidone Pretreatment", Curr. Eye Res. 3, 307-314.)

It has also been shown that certain dopamine analogs of the phenylethylamine class, e.g. N-methyl-dopamine, N,N-dimethyl-dopamine and N,N-di-n-propyldopamine, may alter ocular function by operating through a variety of mechanisms. However, N-methyl dopamine appeared to function by suppressing aqueous humor formation. (See Potter, D.E., Burke, J.A. and Chang, F.W. (1984), "Alteration in Ocular Function Induced by Phenylethylamine Analogs of Dopamine", Curr. Eye Res. 3, 851–859.) Finally, certain aminotetralins were shown to lower intraocular pressure in rabbits. (See Burke, J.A., Chang., F.W. and Potter, D.E. (1984), "Effects of Aminotetralins on Intraocular Pressure and Pupillary Function in Rabbits", J. Auton, Pharmacol. 4, 185–192.)

In EP-A 0 168 505 certain substituted 2-aminotetralins are disclosed. These compounds are useful as dopamine receptor agonists for the treatment of various diseases of the central nervous system such as Parkinson's disease and related disorders.

### Summary of the Invention

There has now been discovered certain novel compounds having the structural formula

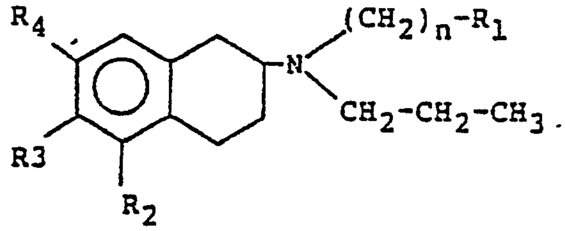

where $R_2$, $R_3$ and $R_4$ are each selected from the group consisting of H and OA; A is H or is selected from the group consisting of hydrocarbyl radicals, e.g. lower alkyl radicals, i.e. methyl, ethyl, propyl or

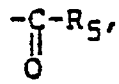

wherein $R_5$ is selected from the group consisting of hydrocarbyl radicals, e.g. alkyl and aromatic residues; n is 2 or 3; and $R_1$ is selected from the group consisting of organic radicals having fused aromatic rings except indole, that is, radicals comprising at least two rings that share a pair of carbon atoms or a carbon and nitrogen atom. Preferably $R_1$ comprises no more than 3 fused aromatic rings, and more preferably $R_1$ comprises 2 fused aromatic rings. Examples of radicals suitable for $R_1$ include naphthyl, anthracyl, phenanthryl, benzofuranyl, benzothienyl, indazolyl, benzotriazolyl, triazolopyridinyl, benzoxazolyl, benzothiazolyl, benzoxadiazoly, benzopyranyl, quinolyl, phthalazinyl. purinyl, naphthothienyl, indolizinyl, quinolizinyl, naphthyridinyl, quinoxalinyl, quinazolinyl, cinnolinyl. The above radicals may also be substituted with various functional groups. In particular, such substituents may be selected from the group consisting of hydroxy, nitro, azido, sulfonamide, halogen, hydrocarbyl and hetero atom-substituted hydrocarbyl radicals, wherein said hetero-atoms are selected from the group consisting of halogen, nitrogen, oxygen, sulfur and phosphorus and said hydrocarbyl radicals comprise from 1 to 12 carbon atoms. In the compounds of the present invention, at least one of $R_2$, $R_3$ and $R_4$ is H, and at least one of $R_2$, $R_3$ and $R_4$ is not H, and $R_2$ and $R_4$ are not both OA.

These compounds are useful as dopamine agonists and, in particular, dopamine D-2 receptor agonists for the treatment of disorders of the central nervous, cardiovascular and endocrine systems such as Parkinson's disease and related disorders, hypertension and hyperprolactinemia.

In particular, the compounds of this invention are useful in the treatment of glaucoma in mammals.

Detailed Description of the Invention

The above compounds may be made by any of the methods disclosed in the patent applications cited above and incorporated by reference herein.

Preferably $R_1$ is selected from the group of radicals represented by the general formulae:

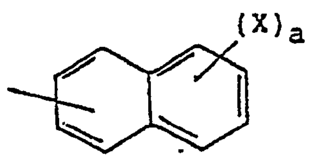

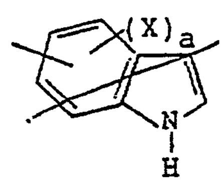

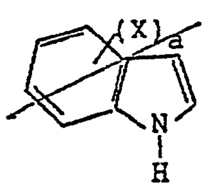

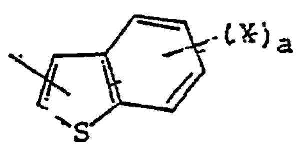

and

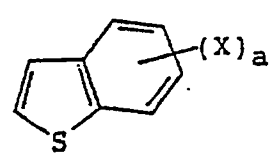

wherein X is selected from the group consisting of hydroxy, alkoxy, nitro, cyano, azido, amino, trifluoromethyl, alkylamino, dialkylamino, halo, carboxyl, carbalkoxy, alkyl, alkenyl, alkynyl, cycloalkyl, aryl, acylamino, carboxamido, sulfonamido, and $SO_m$-(lower)alkyl, wherein m is 0, 1 or 2 and a is an integer of from 0 to 3.

More preferably, X comprises no more than 5 carbon atoms and a is 0 or 1. Specific preferred compounds, which are within the scope of the above general formula include:

2-(N-n-propyl-N-2-[naphthalenyl]ethilamino)-5-hydroxytetralin

2-(N-n-propyl-N-2-[benzothienyl]ethylamino)-5-hydroxytetralin.

2-(N-n-propyl-N-3-[benzothienyl]ethylamino)-5-hydroxytetralin.

A preferred embodiment of this invention is a method of treatment which comprises inducing a dopaminergic response by administering a therapeutically effective amount of one of the foregoing compounds to a patient. In general, a pharmacologically effective daily dose can be from 0.01 mg/kg to 100 mg/kg per day, and preferably from about 0.1 mg/kg to 25 mg/kg per day, bearing in mind, of course, that in selecting the appropriate dosage in any specific case, consideration must be given to the patient's weight, general health, metabolism, age and other factors which influence response to the drug. A particularly preferred dose is 1.0 mg/kg per day.

Another embodiment of this invention is the provision of pharmaceutical compositions in dosage unit form which comprise from about 2 mg. to 500 mg. of a compound of the above formula.

The pharmaceutical composition may be in a form suitable for oral use, for example, as tablets, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents, and preserving agents in order to provide a pharmaceutically elegant and palatable preparation. Tablets contain the active ingredient in admixture with non-toxic pharmaceutically acceptable excipients which are suitable for manufacture of tablets. These excipients may be, for example, inert diluents, for example calcium carbonate, sodium carbonate, lactose, calcium phosphate, or sodium phosphate; granulating and disintegrating agents, for example maize starch, or alginic acid; binding agent, for example starch, gelatine, or acacia; and lubricating agents, for example magnesium stearate, stearic acids, or talc. The tablets may be uncoated or they may be coated by known techniques to delay disintegration and adsorption in the gastrointestinal tract and thereby provide a sustained action over a longer period.

Formulations for oral use may also be presented as hard gelatine capsules wherein the active ingredient is mixed with an inert solid diluent, for example calcium carbonate, calcium phosphate, or kaolin, or as soft gelatine capsules wherein the active ingredient is mixed with an oil medium, for example arachis oil, liquid paraffin, or olive oil.

Aqueous suspensions contain the active compound in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum trangacanth, and gum acacia; dispersing or wetting agents may be a naturally-occurring phosphatide, for example lecithin, or condensation products of an alkylene oxide with fatty acids, for example of polyoxethylene stearate, or condensation products of ethylene oxide with long chain aliphatic alcohols, for example heptadecaethyleneoxycetanol, or condensation products of ethylene oxide with partial esters derived from fatty acids and a hexitol, for example, polyoxyethylene sorbitol monooleate, or condensation product of ethylene oxide with partial esters derived from fatty acids and hexitol anhydrides, for example polyoxyethylene sorbitan monooleate. The said aqueous suspensions may also contain one or more preservatives, for example ethyl, n-propyl, or p-hydroxy benzoate, one or more coloring agents, one or more flavoring agents, and one or more sweetening agents, such as sucrose, saccharin, or sodium or calcium cyclamate.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring, and coloring agents, may also be present.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable preparation, for example as a sterile injectable aqueous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol.

The pharmaceutical compositions may be tableted or otherwise formulated so that for every 100 parts by weight of the composition there are present between 5 and 95 parts by weight of the active ingredient. The dosage unit form will generally contain between about 1 mg. and about 100 mg. of the active ingredient of the formula stated above.

From the foregoing formulation discussion it is apparent that the compositions of this invention can be administered orally or parenterally. The term parenteral as used herein includes subcutaneous injection, intraveneous, intramuscular, or intrasternal injection or fusion techniques.

Even more preferably the compounds of the present invention may be administered to the eye of a mammal to reduce intraocular pressure. Moreover, the levo (–) isomers of these substituted compounds are believed to be the more active.

Suitable ophthalmic carriers are known to those skilled in the art and all such conventional carriers may be employed in the present invention. Thus, a particular carrier may take the form of a sterile ophthalmic ointment, cream, gel, solution, or dispersion and preferably a solution. Also including as suitable ophthalmic carriers are slow releasing polymers, e.g. "Ocusert" polymers, "Hydron" polymers, etc. Stabilizers may also be used as, for example, chelating agents, e.g. EDTA. Anti-oxidants may also be used, e.g. sodium bisulfite, sodium thiosulfite, 8-hydroxy quinoline or ascorbic acid. Sterility typically will be

maintained by conventional ophthalmic preservatives, e.g. chlorbutanol, benzalkonium chloride, cetylpyridinium chloride, phenyl mercuric salts, thimerosal, phenethyl alcohol, etc., for aqueous formulations, and used in amounts which are nontoxic and which generally vary from about 0.001 to about 0.1% by weight of the aqueous solution. Conventional preservatives for ointments include methyl and propyl parabens. Typical ointment bases include white petrolatum and mineral oil or liquid petrolatum. However, preserved aqueous carriers are preferred. Solutions may be manually delivered to the eye in suitable dosage form, e.g., eye drops, or delivered by suitable microdrop or spray apparatus typically affording a metered dose of medicament. Examples of suitable ophthalmic carriers or stabilizers include sterile, substantially isotonic, aqueous solutions containing minor amouns, i.e., less than about 5% by weight hydroxypropylmethylcellulose, polyvinyl alcohol, carboxymethylcellulose, hydroxyethylcellulose, glycerine, EDTA, sodium bisulfite and ascorbic acid.

The amount of active compound to be used in the therapeutic treatment of glaucoma will vary with the age of the patient and the severity of the glaucoma. Generally, a dose level of one or two drops of the foregoing aqueous solution 1-4 times daily would be a suitable dosage amount. Generally, the concentration of active compound will vary between about 0.01 and about 5% and preferably between about 0.05 and about 1%% (wt./v calculated on the basis of the free base) of said ophthalmic composition.

Preferably, the ophthalmic composition of this invention should have a pH within the range of about 4.0 to 9.0 when intended for topical application. Above and below this pH range the solution may irritate and sting the eye of the user. The solutions of the present invention may be maintained between about pH 4.0 and 7.5 with suitable amounts of buffering agents including borate, carbonate, phosphate. Tris (hydroxymethyl aminomethane), acetate and citrate buffers.

A preferred ophthalmic composition is a preserved aqueous solution containing the following ingredients at approximately the indicated concentration.

Table

| | |
|---|---|
| Active compound | 0.001 – 1 wt. % |
| Stabilizer | 0.01 – 0.1 wt. % |
| Preservative | 0.005 – 0.5 wt. % |
| Buffer (sufficient to maintain pH between about 4.0 and 7.5) | 0.1 – 0.001 M |
| NaCl qs. ad. | (isotonic) |
| Water qs. ad. | 100% |

To illustrate the manner in which the invention may be carried out, the following examples are given. It is understood, however, that the examples are for the purpose of illustration and the invention is not to be regarded as limited to any of the specific materials or conditions therein.

Example 1

Preparation of 2-(N-n-propyl-N-2-[naphthyl]ethylamino)-5-hydroxytetralin

A mixture of 2-(N-n-propylamino)-5-methoxytetralin, 2-naphthylacetic acid and trimethylaminoborohydride in dry xylene was refluxed under an atmosphere of nitrogen as described by Horn et al. Pharm. Weekbld. Sci. Ed. 7 208-211, 1985. The resulting methoxy intermediate was demethylated using boron tribromide as described in the above article to yield the desired product.

Example 2

Preparation of 2-(N-n-propyl-N-2-[2-benzothienyl]ethylamino)-5-hydroxytetralin

A mixture of 2-(N-n-propylamino)-5-methoxytetralin, 2-benzothienyl acetic acid and trimethylaminoborohydride in dry xylene is refluxed under an atmosphere of nitrogen as described by Horn et al, Pharm. Weekbld. Sci. Ed. 7 208–211, 1985. The resulting methoxy intermediate is demethylated using boron tribromide as described in the above article to yield the desired product.

Example 3

The pharmacological activity of the product of Example 2 was determined by examining its ability to displace the specific D–2 dopamine receptor binding of a tritium-containing racemic mixture of 2-(N-propyl-N-2thienylethylamino)-5-hydroxytetralin to homogenates of calf brain corpus striatum. In this preparation, which is a modification of the one reported by Mulder et al. "Kinetic and Pharmacological Profiles of the In-Vitro Binding of the Potent Dopamine Agonist $^3$H-N,N-dipropyl-2-Aminotetralin to Rat Striatal

Membranes, "Eur. J. Pharmacol 112 (1985) 73–79, for rat brain corpus striatum, the tritium-containing racemic mixture had an affinity constant ($K_d$) of 1.6 nanomoles and bmax of 26.0 picomoles/gm. The $IC_{50}$ values (i.e. the concentration of drug required to inhibit the binding of labelled drug by 50 percent for the product of Example 2 was 0.56 nanomoles.

The activity at the D-1 dopamine receptor was tested by the method described in Raisman et al. Eur. J.Pharmacol 113: (1985), pp. 467-468, which utilizes membranes isolated from the striatum of bovine brain. In this preparation the compound of Example 2, over a concentration range of from $10^{-11}$ to $10^{-4}$ moles did not displace the tritium labeled SCH 23390 compound, a known, selective D-1 antagonist. Thus, the $IC_{50}$ value for the compound of Example 2 is >100,000 at the D-1 receptor.

The compound of Example 2 thus shows extremely selective D-2 as opposed to D-1 binding, in contrast to dopamine and apomorphine which show a substantially equivalent binding at the D-1 and D-2 receptor.

While particular embodiments of the invention have been described it will be understood, of course, that the invention is not limited thereby since many obvious modifications can be made and it is intended to include within this invention any such modifications as will fall within the scope of the appended claims.

**Claims for the Contracting States: BE, CH, DE, FR, GB, IT, LI, LU, NL, SE**

1. Optically active or racemic compounds having the general formula

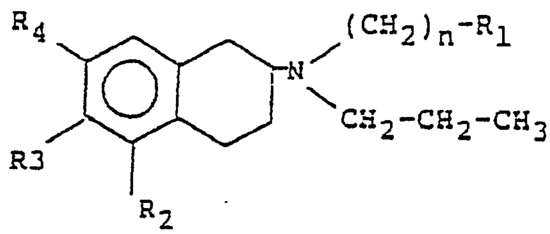

wherein $R_2$, $R_3$ and $R_4$ are each selected from the group consisting of Hand OA; A is selected from the group consisting of hydrogen, hydrocarbyl radicals, and radicals represented by the general formula

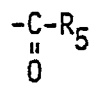

wherein $R_5$ is selected from the group consisting of hydrocarbyl radicals, n is 2 or 3 and $R_1$ is selected from the group consisting of organic radicals having fused aromatic rings, except indole; and wherein at least one of $R_2$, $R_3$ and $R_4$ is H, and at least one of $R_2$, $R_3$ and $R_4$ is not H, and $R_2$ and $R_4$ are not both OA and pharmaceutically-acceptable salts thereof.

2. The compound of claim 1 wherein $R_1$ comprises no more than three fused rings.

3. The compound of claim 2 wherein $R_2$, $R_3$ and $R_4$ are selected from the group consisting of hydrogen and

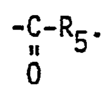

4. The compound of claim 3 wherein $R_5$ is an alkyl or aromatic residue having no more than 20 carbon atoms.

5. The compound of claim 4 wherein $R_1$ is selected from the group consistin of naphthyl, anthracyl, phenanthryl, benzofuranyl, benzothienyl, indazolyl, benzotriazolyl, triazolopyridinyl, benzoxazolyl, benzothiazolyl, benzoxadiazolyl, benzopyranyl, quinolyl, phthalazinyl, purinyl, naphthothienyl, indolizinyl, quinolizinyl, naphthyridinyl, quinoxalinyl, quinazolinyl, cinnolinyl, etc. and substituted derivatives thereof wherein the substituents have no more than 12 carbon atoms and are selected from the group consisting of hydroxy, nitro, azido, sulfonamido, sulfate, halogen, hydrocarbon and heteroatom-substituted hydrocarbon wherein said heteroatoms are selected from the group consisting of halogen, nitrogen, sulfur and phosphorus.

6. The compound of claim 5 wherein $R_1$ is naphthyl.

7. The compound of claim 6 wherein $R_4$ and $R_3$ are hydrogen.

8. The compound of claim 7 wherein A is hydrogen.

9. A composition for reducing the intraocular pressure in mammals which comprises an effective amount of a compound selected from the group consisting of compounds having the structural formula:

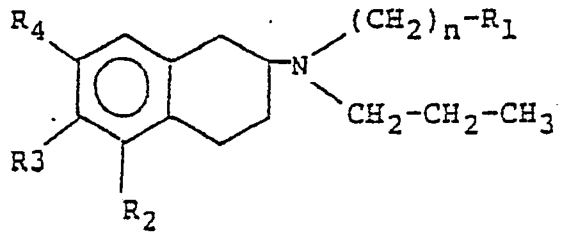

wherein $R_2$, $R_3$ and $R_4$ are each selected from the group consisting of H and OA; A is selected from the group consisting of hydrogen, hydrocarbyl radicals, and radicals represented by the general formula

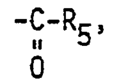

wherein $R_5$ is selected from the group consisting of hydrocarbyl radicals, n is 2 or 3 and $R_1$ is selected from the group consisting of organic radicals having fused aromatic rings, except indole; and wherein at least one of $R_2$, $R_3$ and $R_4$ is H, and at least one of $R_2$, $R_3$ and $R_4$ is not H, and $R_2$ and $R_4$ are not both OA and pharmaceutically acceptable salts thereof, and an ophthalmic carrier for said compound.

10. The composition of claim 9, wherein $R_1$ comprises no more than three fused rings.

11. The composition of claim 10 wherein $R_2$, $R_3$ and $R_4$ are selected from the group consisting of hydrogen and

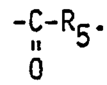

12. The composition of claim 11 wherein $R_5$ is an alkyl or aromatic residue having no more than 20 carbon atoms.

13. The composition of claim 12 wherein $R_1$ is selected from the group consisting of naphthyl, anthracyl, phenanthryl, benzofuranyl, benzothienyl, indazolyl, benzotriazolyl, triazolopyridinyl, benzoxazolyl, benzothiazolyl, benzoxadiazolyl, benzopyranyl, quinolyl, phthalazinyl, purinyl, naphthothienyl, indolizinyl, quinolizinyl, naphthyridinyl, quinoxalinyl, quinazolinyl, cinnolinyl, etc. and substituted derivatives thereof wherein the substituents have no more than 12 carbon atoms and are selected from the group consisting of hydroxy, nitro, azido, sulfonamido, sulfate, halogen, hydrocarbon and heteroatom-substituted hydrocarbon wherein said heteroatoms are selected from the group consisting of halogen, nitrogen, sulfur and phosphorus.

14. The composition of claim 13 wherein $R_5$ is naphthyl.

15. The composition of claim 14 wherein $R_4$ and $R_3$ are hydrogen.

16. The composition of claim 15 wherein A is hydrogen.

**Claims for the Contracting States: AT, GR, ES**

1. A method for preparing a composition for reducing the intraocular pressure in mammals which comprises admixing an effective amount of a compound having the structural formula:

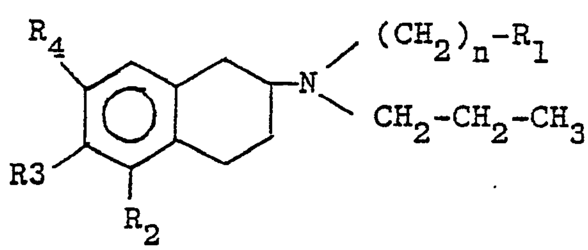

with a non-toxic pharmaceutically acceptable excipients.

2. The method of Claim 1 wherein said composition is a tablet.

3. The method of Claim 1 wherein said composition is a capsule.

4. The method of Claim 1 wherein said composition is an aqueous solution.

5. The method of Claim 1 wherein said composition is dispersible in water.

**Patentansprüche für die Vertragsstaaten: BE, CH, DE, FR, GB, IT, LI, LU, NL, SE**

1. Optisch aktive oder racemische Verbindungen der allgemeinen Formel

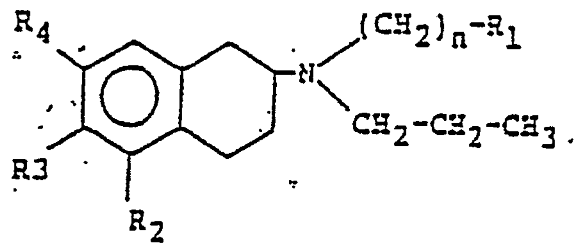

bei denen $R_2$, $R_3$ und $R_4$ aus der aus H und OA bestehenden Gruppe ausgewählt ist; A aus der aus Wasserstoff, Kohlenwasserstoffresten und Resten, die der allgemeinen Formel

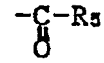

entsprechen ausgewählt ist, in der $R_5$ aus der aus Kohlenwasserstofftresten bestehenden Gruppe ausgewählt ist, n 2 oder 3 ist und $R_1$ aus der Gruppe organischer Reste ausgewählt ist, die kondensierte, aromatische Ringe aufweisen, wobei Indol ausgenommen ist und wobei wenigstens einer der Reste $R_2$, $R_3$ und $R_4$ H ist, wenigstens einer der Reste $R_2$ , $R_3$ und $R_4$ nicht H ist und $R_2$ und $R_4$ nicht beide OA bedeuten, und pharmazeutisch annehmbare Salze davon.

2. Verbindung nach Anspruch 1, bei der $R_1$ nicht mehr als drei kondensierte Ringe enthält.

3. Verbindung nach Anspruch 2, bei der $R_2$, $R_3$ und $R_4$ aus der aus Wasserstoff und

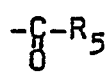

bestehenden Gruppe ausgewählt sind.

4. Verbindung nach Anspruch 3, bei der $R_5$ ein Alkyl oder ein aromatischer Rest mit nicht mehr als 20 Kohlenstoffatomen ist.

5. Verbindung nach Anspruch 4, bei der $R_1$ aus der Gruppe ausgewählt ist, die aus Naphthyl, Anthrazyl, Phenanthryl, Benzofuranyl, Benzothienyl, Indazolyl, Benzotriazolil, .Triazolopyridinyl, Benzoxazolyi, Benzothiazolyl, Benzoxadiazolyl, Benzopyranyl, Quinolyl, Phthalazinyl, Purinyl, Naphthothienyl, Indolizinyl, Quinolizinyl, Naphthyridinyl, Quinoxalinyl, Quinazolinyl, Cinnolinyl, usw. und substituierten Derivaten davon besteht, wobei die Substituenten nicht mehr als 12 Kohlenstoffatome haben und aus der Gruppe ausgewählt sind, die aus Hydroxy, Nitro, Azido, Sulfonamid, Sulfat, Halogen, Kohlenwasserstoffen und Heteroatom-substituierten Kohlenwasserstoffen besteht, wobei die Heteroatome aus der Gruppe ausgewählt sind, die aus Halogenen, Stickstoff, Schwefel und Phosphor besteht.

6. Verbindung nach Anspruch 5, bei der $R_1$ Naphthyl ist.

7. Verbindung nach Anspruch 6, bei der $R_4$ und $R_3$ Wasserstoff sind.

8. Verbindung nach Anspruch 7, bei der A Wasserstoff ist.

9. Zusammensetzung zur Herabsetzung des intraocularen Druckes bei Säugern, die eine wirksame Menge einer Verbindung, die aus der Gruppe von Verbindungen der Strukturformel

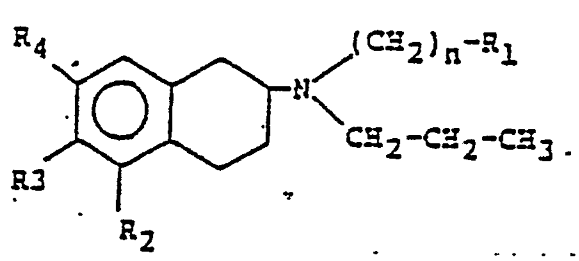

ausgewählt ist, bei der $R_2$, $R_3$ und $R_4$ jeweils aus der Gruppe ausgewählt sind, die aus H und OA besteht; A aus der Gruppe ausgewählt ist, die aus Wasserstoff, Kohlenwasserstoffresten und Resten der allgemeinen Formel

$$-\underset{\overset{\|}{O}}{C}-R_5$$

besteht, wobei $R_5$ aus der Gruppe ausgewählt ist, die aus Kohlenwasserstoffresten besteht, n 2 oder 3 ist und $R_1$ aus der aus organischen Resten mit kondensierten Ringen aufweisenden Gruppe, mit Ausnahme von Indol, ausgewählt ist und wobei wenigstens einer der Reste $R_2$, $R_3$ und $R_4$ H und wenigstens einer der Reste $R_2$, $R_3$ und $R_4$ nicht H ist, $R_2$ und $R_4$ nicht beide OA sind und pharmazeutisch annehmbare Salze davon, und einen ophthalmischen Träger für diese Verbindung enthält.

10. Zusammensetzung nach Anspruch 9, bei der $R_1$ nicht mehr als drei kondensierte Ringe enthält.

11. Zusammensetzung nach Anspruch 10, bei der $R_2$, $R_3$ und $R_4$ aus der Gruppe ausgewählt sind, die aus Wasserstoff und

$$-\underset{\overset{\|}{O}}{C}-R_5$$

besteht.

12. Zusammensetzung nach Anspruch 11, bei der $R_5$ ein Alkyl oder ein aromatischer Rest mit nicht mehr als 20 Kohlenstoffatomen ist.

13. Zusammensetzung nach Anspruch 12, bei der $R_1$ aus der Gruppe ausgewählt ist, die aus Naphthyl, Anthrazyl, Phenanthryl, Benzofuranyl, Benzothienyl, Indazolyl, Benzotriazolyl, Triazolopyridinyl, Benzoxazolyl, Benzothiazolyl, Benzoxadiazolyl, Benzopyranyl, Quinolyl, Phthalazinyl, Purinyl, Naphthothienyl, Indolizinyl, Quinolizinyl, Naphthyridinyl, Quinoxalinyl, Quinazolinyl, Cinnolinyl, usw. und substituierten Derivaten davon besteht, wobei die Substituenten nicht mehr als 12 Kohlenstoffatome haben und aus der Gruppe ausgewählt sind, die aus Hydroxy, Nitro, Azido, Sulfonamid, Sulfat, Halogen, Kohlenwasserstoffen und Heteroatom-substituierten Kohlenwasserstoffen besteht, wobei die Heteroatome aus der Gruppe ausgewählt sind, die aus Halogenen, Stickstoff, Schwefel und Phosphor besteht.

14. Zusammensetzung nach Anspruch 13, bei der $R_5$ Naphthyl ist.

15. Zusammensetzung nach Anspruch 14, bei der $R_4$ und $R_3$ Wasserstoff sind.

16. Zusammensetzung nach Anspruch 15, bei der A Wasserstoff ist.

**Patentansprüche für die Vertragsstaaten: AT, GR, ES**

1. Verfahren zur Herstellung einer Zusammensetzung zur Herabsetzung des intraocularen Druckes bei Säugern, bei dem eine wirksame Menge einer Verbindung der Strukturformel

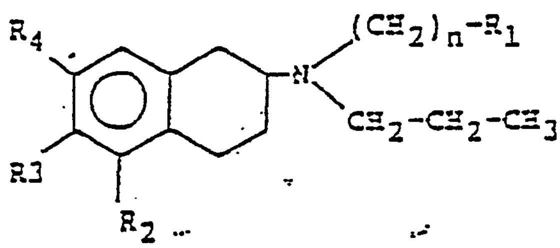

mit nicht toxischen, pharmazeutisch annehmbaren Hüllen oder Bindemitteln versehen bzw. gemischt wird.

2. Verfahren nach Anspruch 1, bei dem die Zusammensetzung eine Tablette ist.

3. Verfahren nach Anspruch 1, bei dem die Zusammensetzung eine Kapsel ist.

4. Verfahren nach Anspruch 1, bei dem die Zusammensetzung eine wässrige Lösung ist.

5. Verfahren nach Anspruch 1, bei dem die Zusammensetzung in Wasser dispergierbar ist.

**Revendications pour les Etats Contractants: BE, CH, DE, FR, GB, IT, LI, LU, NL, SE**

1. Composés optiquement actifs ou racémiques répondant à la formule générale:

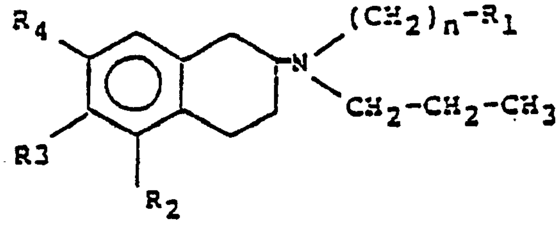

dans laquelle $R_2$, $R_3$ et $R_4$ sont chacun choisis dans le groupe comprenant H et OA, A est choisi dans le groupe comprenant l'hydrogène, les radicaux hydrocarbyle et les radicaux représentés par la formule générale

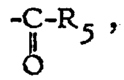

où $R_5$ est choisi dans le groupe comprenant les radicaux hydrocarbyle, n est égal à 2 ou 3 et $R_1$ est choisi dans le groupe comprenant les radicaux organiques comprenant des noyaux aromatiques condensés, à l'exception de l'indole, et dans laquelle au moins l'un des $R_2$, $R_3$ et $R_4$ représente N et au moins l'un des $R_2$, $R_3$ et $R_4$ ne représente pas H, et $R_2$ et $R_4$ ne représentent pas tous deux OA, ainsi que les sels pharmaceutiquement acceptables de ces composés.

2. Composé suivant la revendication 1, caractérisé on ce que $R_1$ ne comprend pas plus de trois noyaux condensés.

3. Composé suivant la revendication 2, caractérisé en ce que $R_2$, $R_3$ et $R_4$ sont choisis dans le groupe comprenant l'hydrogène et

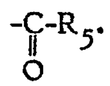

4. Composé suivant la revendication 3, caractérisé en ce que $R_5$ représente un reste d'alkyle ou aromatique ne comportant pas plus de 20 atomes de carbone.

5. Composé suivant la revendication 4, caractérisé on ce que $R_1$ est choisi dans le groupe comprenant les radicaux naphtyle, anthracyle, phénanthryle, benzofurannyle, benzothiényle, indazolyle, benzotriazolyle, triazolopyridinyle, benzoxazolyle, benzothiazolyle, benzoxadiazolyle, benzopyrannyle, quinoléyle, phtalazinyle, purinyle, naphtothiényle, indolizinyle, quinolizinyle, naphtyridinyle, quinoxalinyle, quinazolinyle, clinnolinyle, etc. et leurs dérivés substitués dans lesquels le substituant ne comporte pas plus de 12 atomes de carbone et sont choisis dans le groupe comprenant les radicaux hydroxy, nitro, azido, sulfonamido, sulfate, halogène, hydrocarbonés et hydrocarbonés substitués par des hétéroatomes, dans lesquels ces hétéroatomes sont choisis dans le groupe comprenant les halogènes, l'azote, le soufre et le phosphore.

6. Composé suivant la revendication 5, caractérisé en ce que $R_1$ est un radical naphtyle.

7. Composé suivant la revendication 6, caractérisé en coque $R_4$ et $R_3$ sont de l'hydrogène.

8. Composé suivant la revendication 7, caractérisé en ce que A est de l'hydrogène.

9. Composition pour réduire la tension intraoculaire chez les mammifères, qui comprend une quantité efficace d'un composé choisi dans le groupe comprenant les composés répondant à la formule structurale:

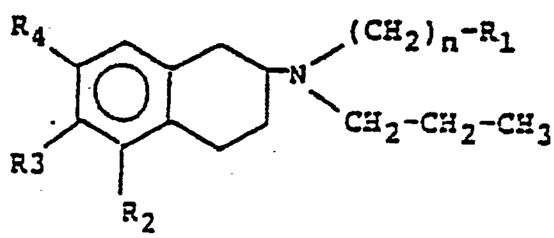

dans laquelle $R_2$, $R_3$ et $R_4$ sont chacun choisis dans le groupe comprenant Net OA, A est choisi dans le groupe comprenant l'hydrogène, les radicaux hydrocarbyle et les radicaux représentés par la formule générale

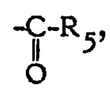

où $R_5$ est choisi dans le groupe comprenant les radicaux hydrocarbyle, n est égal à 2 ou 3 et $R_1$ est choisi dans le groupe comprenant les radicaux organiques comprenant des noyaux aromatiques condensés, à l'exception de l'indole, et dans laquelle au moins l'un des $R_2$, $R_3$ et $R_4$ représente H, et au moins l'un des $R_2$, $R_3$ et $R_4$ ne représente pas N, et $R_2$ et $R_4$ ne représentent pas tous les deux OA, et les sels pharmaceutiquement acceptables de ces composés, ainsi qu'un support ophtalmique pour le composé précité.

10. Composition suivant la revendication 9, caractérisée en ce que $R_1$ ne comprend pas plus de trois noyaux condensés.

11. Composition suivant la revendication 10, caractérisée en ce que $R_2$, $R_3$ et $R_4$ sont choisis dans le groupe comprenant l'hydrogène et

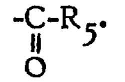

12. Composition suivant la revendication 11, caractérisée en ce que $R_5$ représente un reste d'alkyle ou aromatique ne comportant pas plus de 20 atomes de carbone.

13. Composition suivant la revendication 12, caractérisée en ce que $R_1$ est choisi dans le groupe comprenant les radicaux naphtyle, anthracyle, phénanthryle, benzofurannyle, benzothiényle, indazolyle, benzotriazolyle, triazolopyridinyle, benzoxazolyle, benzothiazolyle, bonzoxadiazolyle, benzopyrannyle, quinoléyle, phtalazinyle, purinyle, naphtothiényle, indolizinyle, quinolizinyle, naphtyridinyle, quinoxalinyle, quinazolinyle, cinnolinyle, etc. et leurs dérivés substitués dans lesquels les substituants ne comportent pas plus de 12 atomes do carbone et sont choisis dans le groupe comprenant les radicaux hydroxy, nitro, azido, sulfonamido, sulfate, halogène, hydrocarbonés et hydrocarbonés substitués par des hétéroatomes dans lesquels les hétéroatomes sont choisis dans le groupe comprenant les halogènes, l'azoto, le soufre et le phosphore.

14. Composition suivant la revendication 13, caractérisée en ce que $R_5$ représente un radical naphtyle.

15. Composition suivant la revendication 14, caractérisée en ce que $R_4$ et $R_5$ représentent de l'hydrogène.

16. Composition suivant la revendication 15, caractérisée en ce que A représente de l'hydrogène.

**Revendications pour les Etats Contractants: AT, ES, GR**

1. Procédé de préparation d'une composition pour réduire la tension intraoculaire chez les mammifère qui comprend le mélange d'une quantité efficace d'un composé répondant à la formule structurale:

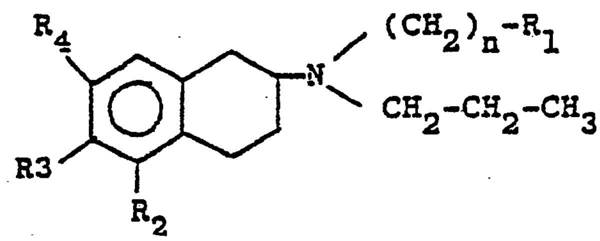

avec un excipient pharmaceutiquement acceptable, non toxique.

2. Procédé suivant la revendication 1, caractérisé en ce que la composition est un comprimé.

3. Procédé suivant la revendication 1, caractérisé en ce que la composition est une capsule.

4. Procédé suivant la revendication 1, caractérisé en ce que la composition est une solution aqueuse.

5. Procédé suivant la revendication 1, caractérisé en ce que la composition est dispersable dans l'eau.